(19) Europäisches Patentamt
European Patent Office
Office européen des brevets

(11) EP 1 776 147 B1

(12) EUROPÄISCHE PATENTSCHRIFT

(45) Veröffentlichungstag und Bekanntmachung des Hinweises auf die Patenterteilung:
**09.09.2015 Patentblatt 2015/37**

(21) Anmeldenummer: **05782591.1**

(22) Anmeldetag: **28.07.2005**

(51) Int Cl.:
***A61L 15/60*** *(2006.01)*

(86) Internationale Anmeldenummer:
**PCT/EP2005/008198**

(87) Internationale Veröffentlichungsnummer:
**WO 2006/015729 (16.02.2006 Gazette 2006/07)**

(54) **VERFAHREN ZUR NACHVERNETZUNG WASSERABSORBIERENDER POLYMERE MIT ZYKLISCHEN CARBAMATEN UND/ODER ZYKLISCHEN HARNSTOFFEN**

METHOD FOR THE SECONDARY CROSSLINKING OF WATER-ABSORBING POLYMERS WITH CYCLIC CARBAMATES AND/OR CYCLIC UREAS

PROCEDE DE POST-RETICULATION DE POLYMERES ABSORBANT L'EAU AVEC DES CARBAMATES CYCLIQUES ET/OU DES UREES CYCLIQUES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **04.08.2004 DE 102004038015**

(43) Veröffentlichungstag der Anmeldung:
**25.04.2007 Patentblatt 2007/17**

(73) Patentinhaber: **BASF SE**
**67056 Ludwigshafen (DE)**

(72) Erfinder:
- **EXNER, Kai Michael**
  **69214 Eppelheim (DE)**
- **DANIEL, Thomas**
  **67165 Waldsee (DE)**
- **ELLIOTT, Mark**
  **67063 Ludwigshafen (DE)**
- **RIEGEL, Ulrich**
  **66849 Landstuhl (DE)**

(56) Entgegenhaltungen:
**DE-A1- 10 204 937**
**DE-A1- 19 807 502**
**DE-A1- 19 854 573**
**US-A- 2 755 286**

- **DATABASE WPI Section Ch, Week 198608 Derwent Publications Ltd., London, GB; Class A60, AN 1986-052867 XP002361502 & JP 61 007260 A (SANYO CHEM IND LTD) 13. Januar 1986 (1986-01-13) in der Anmeldung erwähnt**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein Verfahren zur Nachvernetzung von wasserabsorbierenden Polymeren mit zyklischen Carbamaten und/oder zyklischen Harnstoffen.

Wasserabsorbierende Polymere sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate. Solche Hydrogele werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Zur Verbesserung der Anwendungseigenschaften, wie beispielsweise Flüssigkeitsweiterleitung (SFC) in der Windel und Absorption unter Druck (AUL), werden wasserabsorbierende Polymere im allgemeinen oberflächen- oder gelnachvernetzt. Diese Nachvernetzung erfolgt bevorzugt in wässriger Gelphase oder als Nachvernetzung der gemahlenen und abgesiebten Polymerpartikel.

Dazu geeignete Vernetzer sind Verbindungen, die mindestens zwei Gruppen enthalten, die mit den Carboxylatgruppen des hydrophilen Polymeren kovalente Bindungen bilden können. Geeignete Verbindungen sind beispielsweise Di- oder Polyglycidylverbindungen, wie Phosphonsäurediglycidylester, Alkoxysilylverbindungen, Polyaziridine, Polyamine oder Polyamidoamine, wobei die genannten Verbindungen auch in Mischungen untereinander verwendet werden können (siehe beispielsweise EP-A-0 083 022, EP-A-0 543303 und EP-A-0 530 438). Als Vernetzer geeignete Polyamidoamine sind insbesondere in EP-A-0 349 935 beschrieben.

Ein wesentlicher Nachteil dieser Vernetzer ist deren hohe Reaktivität, da diese besondere Schutzvorkehrungen im Produktionsbetrieb erforderlich macht, um unerwünschte Nebeneffekte zu vermeiden. Ebenso besitzen die vorgenannten Vernetzer hautreizende Eigenschaften, was bei der Verwendung in Hygieneartikeln problematisch erscheint.

Als Vernetzer sind auch polyfunktionelle Alkohole bekannt. Beispielsweise lehren die US-4,666,983 sowie US-5,385,983 die Verwendung von hydrophilen Polyalkoholen bzw. die Verwendung von Polyhydroxytensiden. Die Reaktion wird hiernach bei hohen Temperaturen von 120 bis 250°C durchgeführt. Das Verfahren hat den Nachteil, daß die zur Vernetzung führende Veresterungsreaktion selbst bei diesen Temperaturen nur langsam abläuft.

Desweiteren sind als Vernetzer in DE-A-198 07 502 Oxazolidin-2-on und dessen Derivate, in WO-A-03/031482 Morpholin-2,3-dion und dessen Derivate, in DE-A-198 54 573 2-Oxotetrahydro-1,3-oxazin und dessen Derivate, in DE-A-198 54 574 N-Acyloxazolidin-2-one und in DE-A-198 07 992 Bis- und Polyoxazolidin-2-one als geeignete Vernetzer beschrieben. Diese erfüllen zwar die Anforderungen hinsichtlich einer Verwendung in Hygieneartikeln, sind aber als Verbindungen kommerziell nicht erhältlich und relativ schwierig in Reinform herstellbar.

DE 102 04 937 lehrt die Verwendung von Di- oder Trimethylenharnstoff, der auch N-alkyliert sein kann, als Nachvernetzer für wasserabsorbierende Polymere.

So wird in Tetrahedron Letters, Jahrgang 1974, Seiten 2899 bis 2900, beschrieben, dass N-(2-Hydroxyethyl)-oxazolidin-2-on durch oxidative Carbonylierung von Diethanolamin in Gegenwart von elementarem Selen erhalten werden kann. Für den Einsatz im Hygienebereich ist derartiges Material aufgrund der Giftigkeit von Selen nicht geeignet. Journal of Organic Chemistry, Jahrgang 1961, Seiten 3495 bis 3498, lehrt, dass Diethanolamin mit 92% Ausbeute durch Umsetzung mit Trichloressigsäureethylester in N-(2-Hydroxyethyl)-oxazolidin-2-on überführt werden kann. Diese Variante ist aufgrund der Stoffkosten und dem Anfall von Chloroform als Koppelprodukt nicht wirtschaftlich.

US-2,437,390 offenbart die Herstellung von Oxazolidin-2-onen durch Umsetzung von Ethanolaminen mit Dialkylkarbonaten. Die Dialkylkarbonate werden im Überschuss eingesetzt. Vorzugsweise wird der freigesetzte Alkohol kontinuierlich abdestilliert. Die Oxazolidin-2-one werden destilliert oder umkristallisiert. Nachteilig bei diesem Verfahren ist die Verwendung der teuren Dialkylkarbonate. US-4,933,462 beschreibt in Anlehnung an US-2,437,390 die Verwendung von Dimethylkarbonat.

EP-A-0 900 825 beschreibt die Verwendung von Oxazolidin-2-on als Vernetzer, wobei Oxazolidin-2-on durch Umsetzung von Ethanolamin mit Kaliumcyanat hergestellt wurde.

US-2,755,286 lehrt die Herstellung von Oxazolidin-2-onen durch zyklisierende Umesterung von N-(Hydroxyalkyl)-hydroxyalkylcarbamaten. Die Reaktionsprodukte werden kontinuierlich abdestilliert und die Oxazolidin-2-one kristallisieren im Destillat. Zur Destillation der Oxazolidin-2-one sind aufwendige Destillationen bei Drücken von 1 bis 1,2 Torr erforderlich, die in der Technik nur zu sehr hohen Kosten erreicht werden können. Destillationen bei höheren Drücken, die mit höheren Temperaturen einhergehen, führen zu polymeren Folgeprodukten, wie in Nippon Kagaku Kaishi, Jahrgang 1974, Band 8, Seiten 1592 bis 1594, beschrieben. Die N-(Hydroxyalkyl)-hydroxyalkylcarbamate können durch Umsetzung von 2-Hydroxyalkylaminen mit zyklischen Karbonaten erhalten werden.

JP-61-007260 offenbart die Herstellung von Oxazolidin-2-onen durch Umsetzung von Ethanolaminen mit zyklischen Karbonaten bei vermindertem Druck, wobei das entstehende Glykol destillativ entfernt werden kann.

JP-60-097967 und JP-60-152476 beschreiben die Umsetzung von Ethanolaminen mit zyklischen Karbonaten, wobei die Reaktionsprodukte mittels saurer Ionenaustauscherharze gereinigt werden.

JP-2002-105064 beschreibt die Umsetzung von Dialkylkarbonaten mit Ethanolaminen in Gegenwart von Alkalimetallalkoxiden.

Es bestand daher die Aufgabe ein Verfahren zur Nachvernetzung wasserabsorbierender Polymere bereitzustellen, welches Nachvernetzer verwendet, die arbeitshygienisch unbedenklich sind aber gleichzeitig eine ausreichende Reaktivität aufweisen sowie einfach herzustellen sind. Insbesondere sollten die Nachvernetzer bei der Herstellung in einer Form anfallen, dass auf eine Reinigung der Rohprodukte, beispielsweise durch Destillation oder Kristallisation der Nachvernetzer verzichtet werden kann.

Eine weitere Aufgabe bestand darin eine Nachvernetzermischung bereitzustellen, die eine hohe Lagerstabilität aufweist.

Die Nachvernetzer dürfen im Endprodukt auch nicht, beispielsweise durch Neben- oder Folgeprodukte, zu Verfärbungen oder Gerüchen führen.

Gelöst wurde die Aufgabe durch die Bereitstellung eines Verfahren zur Nachvernetzung wasserabsorbierender Polymere, indem das Polymer mit mindestens einem Nachvernetzer behandelt wird, wobei der mindestens eine Nachvernetzer ein zyklisches Carbamat oder ein zyklischer Harnstoff ist, dadurch gekennzeichnet, dass das zyklische Carbamat oder der zyklische Harnstoff durch Umsetzung eines Aminoalkohols bzw. eines Diamins mit einem zyklischen Karbonat erhalten, aus dem zyklischen Karbonat ein k,m-Diol freigesetzt wurde, wobei gilt $m > n$ und $m - n > 1$, und das n,m-Diol nach beendeter Reaktion zu höchstens 40% abdestilliert wird.

Vorzugsweise werden die wasserabsorbierenden Polymere während oder nach dem Behandeln mit dem mindestens einem Nachvernetzer durch Temperaturerhöhung nachvernetzt und/oder getrocknet.

Besonders bevorzugt werden die wasserabsorbierenden Polymere während oder nach dem Behandeln mit dem mindestens einem Nachvernetzer durch Temperaturerhöhung getrocknet und nachvernetzt.

Vorzugsweise werden zyklische Carbamate eingesetzt. Geeignete zyklische Carbamate sind beispielsweise Verbindungen der allgemeinen Formel I

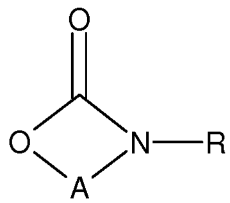

(I)

worin

R Wasserstoff, $C_1$-$C_{12}$-Alkyl, $C_2$-$C_{12}$-Alkenyl, $C_2$-$C_{12}$-Acyl oder $C_2$-$C_{12}$-Hydroxyalkyl bedeuten, wobei $C_3$-$C_{12}$-Alkyl, $C_3$-$C_{12}$-Alkenyl, $C_3$-$C_{12}$-Acyl oder $C_3$-$C_{12}$-Hydroxyalkyl verzweigt oder unverzweigt und $C_1$-$C_{12}$-Alkyl, $C_2$-$C_{12}$-Alkenyl, $C_2$-$C_{12}$-Acyl oder $C_2$-$C_{12}$-Hydroxyalkyl halogensubstituiert sein können sowie Halogen Fluor, Chlor, Brom und/oder Jod bedeutet, und

A $C_2$-$C_{12}$-Alkylen bedeutet, wobei $C_3$-$C_{12}$-Alkylen verzweigt oder unverzweigt und $C_2$-$C_{12}$-Alkylen halogensubstituiert sein können sowie Halogen Fluor, Chlor, Brom und/oder Jod bedeutet,

ist.

Beispiele für Reste R sind Wasserstoff, Methyl, Ethyl, Propyl, Prop-2-yl (Isopropyl), Butyl, But-2-yl (Isobutyl), Pentyl, Pent-2-yl, Pent-3-yl, 2-Methylpentyl, 3-Methylpentyl, 3-Methylpent-2-yl, 2,2-Dimethylpropyl, Trifluormethyl, Ethenyl, Propen-2-yl, Buten-2-yl, Acetyl, 2-Hydroxyethyl, 2-Hydroxypropyl, 3-Hydroxypropyl, 2-Hydroxybutyl, 3-Hydroxybutyl, 4-Hydroxybutyl, 2-Hydroxy-2-methylpropyl und 3-Hydroxy-2-methylpropyl.

Beispiele für Reste A sind

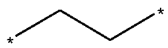

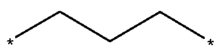

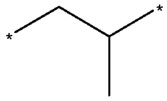
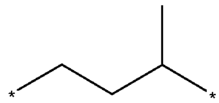

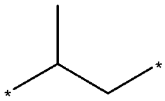
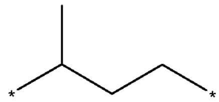

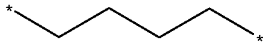
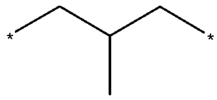

wobei mit * die Verknüpfungspositionen gekennzeichnet sind.

Bevorzugt sind zyklische Carbamate der allgemeinen Formel I, in der R Wasserstoff, $C_1$-$C_6$-Alkyl, $C_2$-$C_6$-Alkenyl, $C_2$-$C_6$-Acyl oder $C_2$-$C_6$-Hydroxyalkyl bedeuten, wobei $C_3$-$C_6$-Alkyl, $C_3$-$C_6$-Alkenyl, $C_3$-$C_6$-Acyl oder $C_3$-$C_6$-Hydroxyalkyl verzweigt oder unverzweigt und $C_1$-$C_6$-Alkyl, $C_2$-$C_6$-Alkenyl, $C_2$-$C_6$-Acyl oder $C_2$-$C_6$-Hydroxyalkyl halogensubstituiert sein können sowie Halogen Fluor, Chlor, Brom und/oder Jod bedeutet, und A $C_2$-$C_6$-Alkylen bedeutet, wobei $C_3$-$C_6$-Alkylen verzweigt oder unverzweigt und $C_2$-$C_6$-Alkylen halogensubstituiert sein können sowie Halogen Fluor, Chlor, Brom und/oder Jod bedeutet.

Besonders bevorzugt sind zyklische Carbamate der allgemeinen Formel I, in der R Wasserstoff, $C_2$-$C_6$-Hydroxyalkyl bedeuten, wobei $C_3$-$C_6$-Hydroxyalkyl verzweigt oder unverzweigt sein können, und A $C_2$-$C_6$-Alkylen bedeutet, wobei $C_3$-$C_6$-Alkylen verzweigt oder unverzweigt sein können.

Ganz besonders bevorzugt sind zyklische Carbamate der allgemeinen Formel I, in der R Wasserstoff, $C_2$-$C_3$-Hydroxyalkyl bedeuten, wobei $C_3$-Hydroxyalkyl verzweigt oder unverzweigt sein kann, und A $C_2$-$C_3$-Alkylen bedeutet, wobei $C_3$-Alkylen verzweigt oder unverzweigt sein kann.

Ganz besonders bevorzugte Reste R sind 2-Hydroxyethyl, 2-Hydroxypropyl und 3-Hydroxypropyl.

Die Verbindung der allgemeinen Formel I ist vorzugsweise ein Fünf-oder Sechsring.

Bevorzugte zyklische Carbamate sind Oxazolidin-2-on, 2-Hydroxyethyloxazolidin-2-on, 3-Hydroxyethyloxazolidin-2-on, 2-Hydroxypropyloxazolidin-2-on und 2-Oxotetrahydro-1,3-oxazin.

Die zyklischen Carbamate werden durch thermische Umsetzung eines Aminoalkohols mit einem zyklischen Karbonat hergestellt. Geeignete zyklische Karbonate sind beispielsweise 1,3-Dioxan-2-on, 4-Methyl-1,3-dioxan-2-on, 5-Methyl-1,3-dioxan-2-on, 5,5-Dimethyl-1,3-dioxan-2-on, 4,6-Dimethyl-1,3-dioxan-2-on und 1,3-Dioxepan-2-on. Geeignete Aminoalkohole sind beispielsweise Ethanolamin, Diethanolamin und N-Hydroxyethylpropanolamin. Die Reaktionstemperatur beträgt üblicherweise 80 bis 200°C, vorzugsweise 100 bis 150°C, besonders bevorzugt 110 bis 130°C. Die Reaktion kann bei Unterdruck, Überdruck oder Atmosphärendruck durchgeführt werden, letzteres ist bevorzugt. Vorzugsweise wird das bei der Reaktion aus dem zyklischen Karbonat freigesetzte Diol während der Reaktion nicht abdestilliert.

Die Reaktion kann natürlich beschleunigt werden, indem das bei der Reaktion freigesetzte Diol kontinuierlich abdestilliert wird, vorzugsweise bei einem der gewünschten Reaktionstemperatur entsprechenden Druck.

Vorteilhaft werden basische Katalysatoren verwendet, beispielsweise Natriumhydroxid, Kaliumhydroxid, Natriummethylat, Natriumkarbonat und Kaliumkarbonat. Dadurch werden auch bei niedrigeren Reaktionstemperaturen ausreichend hohe Reaktionsgeschwindigkeiten erreicht und Produktverfärbungen minimiert. Bei Verwendung basischer Katalysatoren beträgt die Reaktionstemperatur üblicherweise 60 bis 180°C, vorzugsweise 70 bis 140°C, besonders bevorzugt 90 bis 110°C.

Dialkanolamine, wobei die Alkanol-Gruppen gleich oder verschieden sein können, reagieren nahezu quantitativ und sind daher als Einsatzstoff bevorzugt. Geeignete Dialkanolamine sind N,N-Di-(2-ethanol)-amin, N,N-Di-(2-propanol)-amin, N,N-Di-(3-propanol)-amin, N,N-Di-(2-butanol)-amin, N,N-Di-(3-butanol)-amin, N-Ethanol-N-(2-propanol)-amin, N-(2-Ethanol)-N-(2-butanol)-amin und N-(2-propanol)-N-(2-butanol)-amin. N,N-Di-(2-ethanol)-amin oder Diethanolamin ist bevorzugt.

Das bei der Reaktion anfallende Rohprodukt ist farblos und enthält keine riechenden Neben- oder Folgeprodukte und kann daher direkt für die Nachvernetzung verwendet werden.

Das bei der Reaktion aus dem zyklischen Karbonat freigesetzte n,m-Diol, wobei gilt m > n und m-n > 1, wird nach beendeter Reaktion im wesentlichen nicht abdestilliert, d.h. höchstens zu 40%, vorzugsweise höchstens zu 20%, besonders bevorzugt höchtens zu 10%.

Das zyklische Carbamat wird üblicherweise nicht weiter gereinigt, also nicht destilliert oder umkristallisiert, insbesondere wenn das als Nebenprodukt entstehende Diol weder die Nachvernetzung beeinträchtigt noch zu Geruchsproblemen führt.

Analog zu den zyklischen Carbamaten können auch zyklische Harnstoffe hergestellt und eingesetzt werden.

Das zyklische Carbamat wird vorzugsweise in inerten Lösungsmitteln gelöst eingesetzt. Als inertes Lösemittel bevorzugt ist Wasser sowie Gemische von Wasser mit Alkoholen. Es können jedoch alle mit Wasser unbegrenzt mischbaren organischen Lösemittel eingesetzt werden, die nicht selbst unter den Verfahrensbedingungen reaktiv sind. Sofern ein Alkohol/Wasser-Gemisch eingesetzt wird, beträgt der Alkoholgehalt dieser Lösung beispielsweise 10 bis 90 Gew.-%, bevorzugt 15 bis 70 Gew.-%, insbesondere 20 bis 60 Gew.-%. Es können alle mit Wasser unbeschränkt mischbaren Alkohole eingesetzt werden, sowie Gemische mehrerer Alkohole (beispielsweise Methanol + Isopropanol + Wasser). Die Alkoholgemische können die Alkohole in beliebigem Mischungsverhältnis enthalten. Besonders bevorzugt ist jedoch der Einsatz folgender Alkohole in wässriger Lösung: Methanol, Ethanol, n-Propanol, Polyethylenglykole mit bis zu 20 EO-Einheiten, Propan-1,3-diol, 2-Methylpropan-1,3-diol, 2,2-Dimethylpropan-1,3-diol, Butan-1,4-diol und besonders bevorzugt Isopropanol.

Selbstverständlich kann der Nachvernetzer als nichtwässrige Lösung eingesetzt werden. In diesem Fall wird das getrocknete Hydrogel vor, während oder nach dem Aufsprühen der Nachvernetzerlösung mit Wasser angefeuchtet.

Im erfindungsgemäßen Verfahren können die zyklischen Carbamate allein oder in Kombination mit anderen Nachvernetzern verwendet werden, beispielsweise Ethylenglykoldiglycidylether, Diethylenglykoldiglycidylether, Polyethylenglykoldiglycidylether, Propylenglykoldiglycidylether, Dipropylenglykoldiglycidylether, Polypropylenglykoldiglycidylether, Glycerindiglycidylether, Polyglycerindiglycidylether, Epichlorhydrin, Etylendiamin, Diethylenglykol, Triethylenglykol, Polyethylenglykol, Dipropylenglykol, Tripropylenglykol, Polypropylenglykol, 1,3-Propandiol, 1,4-Butandiol, Bisphenol A, Trimethylolpropan, Pentaerythrit, Diethanolamin, Triethanolamin, Etylendiamin.

Bevorzugt können auch zusätzlich Salze polyvalenter Kationen bei der Nachvernetzung verwendet werden. Polyvalente Kationen sind beispielsweise zweiwertige Kationen, wie die Kationen von Zink, Magnesium, Kalzium, Barium und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat und Lactat, möglich. Aluminiumsulfat ist bevorzugt. Weiterhin bevorzugt sind Dispersionen von Kalziumphosphat, Kalziumsulfat und Bariumsulfat.

In einer bevorzugte Ausführungsform wird das zyklische Carbamat zusammen mit einem Diol eingesetzt. Geeignete Diole sind $C_3$-$C_{12}$-Diole, die verzweigt oder unverzweigt sein können. Vorteilhaft verbleibt das bei der Herstellung des zyklischen Carbamats aus dem zyklischen Karbonat freigesetzte Diol zumindest teilweise im Rohprodukt, dass dann vorzugsweise direkt, ohne weitere Reinigung, eingesetzt werden kann.

Bevorzugte Diole sind sind $C_3$-$C_6$-Diole, die verzweigt oder unverzweigt sein können.

Verwendet werden Diole, die keine Glykole (vincinale Diole) sind, wie Propan-1,3-diol, Butan-1,3-diol, 2-Methylpropan-1,3-diol 2,2-Dimethylpropan-1,3-diol und Butan-1,4-diol.

Glykole reagieren unter den Bedingungen der Nachvernetzung zumindest teilweise zu riechenden Folgeprodukten, beispielsweise entsteht aus Propylenglykol bei 160°C Aceton, welches bei Befeuchtung des Superabsorbers freigesetzt wird und zu unangenehmen Gerüchen führt.

In einer besonders bevorzugten Ausführungsform wird das zyklische Carbamat zusammen mit einem organischen Karbonat, vorzugsweise einem zyklischen Karbonat, eingesetzt. Geeignete zyklische Karbonate sind beispielsweise 1,3-Dioxan-2-on, 4-Methyl-1,3-dioxan-2-on, 5-Methyl-1,3-dioxan-2-on, 5,5-Dimethyl-1,3-dioxan-2-on, 4,6-Dimethyl-1,3-dioxan-2-on und 1,3-Dioxepan-2-on. Vorteilhaft wird bei der Herstellung des zyklischen Carbamats ein Überschuss an zyklischem Karbonat eingesetzt, das dann zumindest teilweise im Rohprodukt verbleibt, vorzugsweise nahezu vollständig.

Die Aufbringung der Nachvernetzungslösung erfolgt bevorzugt durch Aufsprühen auf das Polymer in geeigneten Sprühmischern. Im Anschluß an das Aufsprühen wird das Polymerpulver thermisch getrocknet, wobei die Vernetzungsreaktion sowohl vor als auch während der Trocknung stattfinden kann. Bevorzugt ist das Aufsprühen einer Lösung des Vernetzers in Reaktionsmischern oder Misch- und Trocknungsanlagen wie beispielsweise Lödige-Mischer, BEPEX®-Mischer, NAUTA®-Mischer, SCHUGI®-Mischer oder PROCESSALL®. Überdies können auch Wirbelschichttrockner eingesetzt werden. Bevorzugt zur Nachvernetzung und Trocknung ist dabei der Temperaturbereich von 30 bis 210°C, insbesondere 80 bis 195°C, besonders bevorzugt 120 bis 185°C.

Die Trocknung kann im Mischer selbst erfolgen, durch Beheizung des Mantels oder Einblasen von Warmluft. Ebenso geeignet ist ein nachgeschalteter Trockner wie ein Hordentrockner, ein Drehrohrofen, oder eine beheizbare Schnecke. Es kann aber auch beispielsweise eine azeotrope Destillation als Trocknungsverfahren benutzt werden. Die bevorzugte Verweilzeit bei dieser Temperatur im Reaktionsmischer oder Trockner beträgt unter 120 Minuten, besonders bevorzugt unter 90 Minuten und am meisten bevorzugt unter 60 Minuten.

Das zyklische Carbamat wird dabei in einer Menge von 0,01 bis 5 Gew.-%, bevorzugt 0,01 bis 1,0 Gew.-%,

vorzugsweise 0,05 bis 0,5 Gew.-%, bezogen auf das eingesetzte Polymer, verwendet.

Das polyvalente Kation wird in einer Menge von 0 bis 0,5 Gew.-%, vorzugsweise 0,005 bis 0,1 Gew.-%, besonders bevorzugt 0,03 bis 0,08 Gew.-%, bezogen auf das eingesetzte Polymer, verwendet.

Wird das zyklische Carbamat zusammen mit einem Diol eingesetzt, so beträgt das Molverhältnis Diol zu Carbamat üblicherweise von 1:50 bis 2:1, vorzugsweise von 1:10 bis 1,5:1, besonders bevorzugt von 1:2,5 bis 1,1:1.

Wird das zyklische Carbamat zusammen mit einem organischen Karbonat eingesetzt, so beträgt das Molverhältnis Carbamat zu Karbonat üblicherweise von 1000:1 bis 2:1, vorzugsweise von 200:1 bis 5:1, besonders bevorzugt von 40:1 bis 10:1.

In einer ganz besonders bevorzugten Ausführungsform wird eine Nachvernetzerlösung eingesetzt, d.h., die Nachvernetzerlösung enthält neben dem zyklischen Carbamat alle weiteren Komponenten, wie Lösungsmittel, weitere Nachvernetzer, polyvalente Kationen, Diol, Tenside, wie Sorbitanmonolaurat, dispergierbare anorganische Pulver, wie pyrogene Kieselsäure und Kalziumphoshat, und/oder organische Karbonate.

Die organischen Karbonate erhöhen insbesondere die Lagerstabilität der Nachvernetzerlösung.

In einer weiteren bevorzugten Ausführungsform der Erfindung wird die Nachvernetzerlösung in einem Verhältnis von 0,5 bis 20 Gew.-%, bezogen auf die Masse des Polymeren eingesetzt. Besonders bevorzugt ist eine Lösungsmenge von 1 bis 10 Gew.-%, bezogen auf das Polymer.

Die im erfindungsgemäßen Verfahren einzusetzenden wasserabsorbierenden Polymere sind insbesondere Polymere aus vernetzten (co)polymerisierten hydrophilen Monomeren, Polyasparaginsäure, Pfropf(co)polymere von einem oder mehreren hydrophilen Monomeren auf eine geeignete Pfropfgrundlage, vernetzte Cellulose- oder Stärkeether oder in wässrigen Flüssigkeiten quellbare Naturprodukte, wie beispielsweise Guarderivate. Bevorzugt handelt es sich bei dem zu vernetzenden Polymer um ein Polymer, das Struktureinheiten enthält, die sich von Acrylsäure oder deren Estern ableiten, oder die durch Pfropfcopolymerisation von Acrylsäure oder Acrylsäureestern auf eine wasserlösliche Polymermatrix erhalten wurden. Diese Hydrogele sind dem Fachmann bekannt und beispielsweise in der US-4 286 082, DE-C-27 06 135, US-A-4 340 706, DE-C-37 13 601, DE-C-28 40 010, DE-A-43 44 548, DE-A-40 20 780, DE-A-40 15 085, DE-A-39 17 846, DE-A-38 07 289, DE-A-35 33 337, DE-A-35 03 458, DE-A-42 44 548, DE-A-42 19 607, DE-A-40 21 847, DE-A-38 31 261, DE-A-35 11 086, DE-A-31 18 172, DE-A-30 28 043, DE-A-44 18 881, EP-A-0 801 483, EP-A-0 455 985, EP-A-0 467 073, EP-A-0 312 952, EP-A-0 205 874, EP-A-0 499 774, DE-A 26 12 846, DE-A-40 20 780, EP-A-0 205 674, US-A-5 145 906, EP-A-0 530 438, EP-A-0 670 073, US-A-4 057 521, US-A-4 062 817, US-A-4 525 527, US-A-4 295 987, US-A-5 011 892, US-A-4 076 663 oder US-A-4 931 497 beschrieben.

Zur Herstellung dieser wasserabsorbierenden Polymer geeignete hydrophile Monomere sind beispielsweise polymerisationsfähige Säuren, wie Acrylsäure, Methacrylsäure, Vinylsulfonsäure, Vinylphosphonsäure, Maleinsäure einschließlich deren Anhydrid, Fumarsäure, Itaconsäure, 2-Acrylamido-2-methylpropansulfonsäure, 2-Acrylamido-2-methylpropanphosphonsäure sowie deren Amide, Hydroxyalkylester und aminogruppen- oder ammoniumgruppenhaltige Ester und Amide sowie die Alkalimetall- und/oder Ammoniumsalze der Säuregruppen enthaltenden Monomeren. Des weiteren eignen sich wasserlösliche N-Vinylamide wie N-Vinylformamid oder auch Diallyldimethyl-ammoniumchlorid. Bevorzugte hydrophile Monomere sind Verbindungen der allgemeinen Formel II

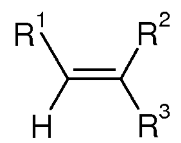

(II)

worin

$R^1$ Wasserstoff, Methyl, Ethyl oder Carboxyl,

$R^2$ -COOR$^4$, Hydroxysulfonyl oder Phosphonyl, eine mit einem $C_1$-$C_4$-Alkanol veresterte Phosphonylgruppe oder eine Gruppe der Formel III

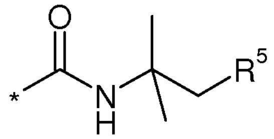

(III)

$R^3$ Wasserstoff, Methyl oder Ethyl,

$R^4$ Wasserstoff, $C_1$-$C_4$-Aminoalkyl, $C_1$-$C_4$-Hydroxyalkyl, Alkalimetall- oder Ammoniumion und

$R^5$ eine Sulfonylgruppe, eine Phosphonylgruppe oder eine Carboxylgruppe oder jeweils deren Alkalimetall- oder Ammoniumsalze, bedeuten.

Beispiele für $C_1$-$C_4$-Alkanole sind Methanol, Ethanol, n-Propanol, Isopropanol oder n-Butanol.

Besonders bevorzugte hydrophile Monomere sind Acrylsäure und Methacrylsäure, sowie deren Alkalimetall- oder Ammoniumsalze, beispielsweise Natriumacrylat, Kaliumacrylat oder Ammoniumacrylat.

Geeignete Pfropfgrundlagen für wasserabsorbierende Polymere, die durch Pfropfcopolymerisation olefinisch ungesättigter Säuren oder ihrer Alkalimetall- oder Ammoniumsalze erhältlich sind, können natürlichen oder synthetischen Ursprungs sein. Beispiele sind Stärke, Cellulose oder Cellulosederivate sowie andere Polysaccharide und Oligosaccharide, Polyalkylenoxide, insbesondere Polyethylenoxide und Polypropylenoxide, sowie hydrophile Polyester.

Geeignete Polyalkylenoxide haben beispielsweise die Formel IV

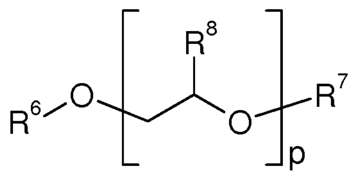

(IV)

worin

$R^6$, $R^7$ unabhängig voneinander Wasserstoff, Alkyl, Alkenyl oder Aryl,

$R^8$ Wasserstoff oder Methyl und

p eine ganze Zahl von 1 bis 500 bedeuten.

$R^6$ und $R^7$ bedeuten bevorzugt Wasserstoff, $C_1$-$C_4$-Alkyl, $C_2$-$C_6$-Alkenyl oder Phenyl.

Bevorzugte wasserabsorbierende Polymere sind insbesondere Polyacrylate, Polymethacrylate sowie die in der US-4,931,497, US-5,011,892 und US-5,041,496 beschriebene Pfropfpolymere.

Die wasserabsorbierenden Polymere sind bevorzugt vernetzt, d.h. sie enthalten Verbindungen mit mindestens zwei Doppelbindungen, die in das Polymernetzwerk einpolymerisiert sind. Geeignete Vernetzer sind insbesondere N,N'-Methylenbisacrylamid und N,N'-Methylenbismethacrylamid, Ester ungesättigter Mono- oder Polycarbonsäuren von Polyolen, wie Diacrylat oder Triacrylat, beispielsweise Butandiol- oder Ethylendiacrylat bzw. -methacrylat sowie Trimethylolpropantriacrylat und Allylverbindungen wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP-A-0 343 427 beschrieben sind. Im erfindungsgemäßen Verfahren einsetzbar sind auch Di(meth)acrylate von Polyethylenglykolen, wobei der eingesetzte Polyethylenglykol ein Molekulargewicht zwischen 300 und 1000 aufweist. Weiterhin geeignet sind Di- und/oder Tri(meth)acrylate des mehrfach ethoxylierten Trimethylolpropans oder Trimethylolethans. Besonders geeignet sind Tri(meth)acrylate des 5-fach bis 30-fach ethoxylierten Trimethylolpropans oder Trimethylolethans. Ganz besonders geeignet sind Tri(meth)acrylate des 10-fach bis 20-fach ethoxylierten Trimethylolpropans oder Trimethylolethans. Am meisten geeignet sind die Triacrylate des 13-fach bis 18-fach ethoxylierten Trimethylolpropans oder Trimethylolethans.

Weiterhin einsetzbar im erfindungsgemäßen Verfahren sind auch Hydrogele, die unter Verwendung von Polyallylethern als Vernetzer und durch saure Homopolymerisation von Acrylsäure hergestellt werden. Geeignete Vernetzer sind Pentaerythritoltri- und - tetraallylether, Polyethylenglykoldiallylether, Monoethylenglykoldiallylether, Glyceroldi- und Triallylether, Polyallylether auf Basis Sorbitol, sowie ethoxylierte Varianten davon.

Ganz besonders bevorzugte Vernetzer sind die mit Acrylsäure oder Methacrylsäure zu Di- oder Triacrylaten veresterten mehrfach ethoxylierten und/oder propoxylierten Glycerine wie sie beispielsweise in WO 03/104 301 A1 beschrieben sind. Besonders vorteilhaft sind Di- und/oder Triacrylate des 3- bis 10-fach ethoxylierten Glycerins. Ganz besonders bevorzugt sind Di- oder Triacrylate des 1- bis 5- fach ethoxylierten und/oder propoxylierten Glycerins. Am meisten bevorzugt sind die Triacrylate des 3- bis 5-fach ethoxylierten oder propoxylierten Glycerins.

Die bevorzugten Herstellverfahren für das im erfindungsgemäßen Verfahren einsetzbare Grundpolymer werden in "Modern Superabsorbent Polymer Technology", F.L. Buchholz and A.T. Graham, Wiley-VCH, 1998, Seiten 77 bis 84 beschrieben. Besonders bevorzugt sind Basispolymere, die im Kneter, wie beispielsweise in WO-A-01/38402 beschrieben, oder auf einem Bandreaktor, wie beispielsweise in EP-A-0 955 086 beschrieben, hergestellt werden.

Das wasserabsorbierende Polymer ist bevorzugt eine polymere Acrylsäure oder ein Polyacrylat. Die Herstellung dieses wasserabsorbierenden Polymeren kann nach einem aus der Literatur bekannten Verfahren erfolgen. Bevorzugt sind Polymere, die vernetzende Comonomere in Mengen von 0,001 bis 10 mol-%, vorzugsweise 0,01 bis 1 mol-% enthalten, ganz besonders bevorzugt sind jedoch Polymere, die durch radikalische Polymerisation erhalten wurden und bei denen ein mehrfunktioneller ethylenisch ungesättigter Radikalvernetzer verwendet wurde, der zusätzlich noch mindestens eine freie Hydroxylgruppe trägt (wie beispielsweise Pentaerythritoltriallylether oder Trimethylolpropandiallylether).

Die wasserabsorbierenden Polymere können durch an sich bekannte Polymerisationsverfahren hergestellt werden. Bevorzugt ist die Polymerisation in wässriger Lösung nach dem Verfahren der sogenannten Gelpolymerisation. Dabei werden beispielsweise 15 bis 50 gew.-%ige wässrige Lösungen eines oder mehrerer hydrophiler Monomere und gegebenenfalls einer geeigneten Pfropfgrundlage in Gegenwart eines Radikalinitiators, bevorzugt ohne mechanische Durchmischung unter Ausnutzung des Trommsdorff-Norrish-Effektes (Makromol. Chem. 1, 169 (1947)), polymerisiert. Die Polymerisationsreaktion kann im Temperaturbereich zwischen 0 und 150°C, vorzugsweise zwischen 10 und 100°C, sowohl bei Normaldruck als auch unter erhöhtem oder erniedrigtem Druck durchgeführt werden. Wie üblich kann die Polymerisation auch in einer Schutzgasatmosphäre, vorzugsweise unter Stickstoff, ausgeführt werden. zur Auslösung der Polymerisation können energiereiche elektromagnetische Strahlen oder die üblichen chemischen Polymerisationsinitiatoren herangezogen werden, beispielsweise organische Peroxide, wie Benzoylperoxid, tert.-Butylhydroperoxid, Methylethylketonperoxid, Cumolhydroperoxid, Azoverbindungen wie Azodiisobutyronitril sowie anorganische Peroxoverbindungen wie $(NH_4)_2S_2O_8$, $K_2S_2O_8$, $Na_2S_2O_8$ oder $H_2O_2$. Sie können gegebenenfalls in Kombination mit Reduktionsmitteln wie Natriumhydrogensulfit und Eisen(II)-sulfat oder Redoxsystemen, welche als reduzierende Komponente eine aliphatische und aromatische Sulfinsäure, wie Benzolsulfinsäure und Toluolsulfinsäure oder Derivate dieser Säuren enthalten, wie Mannichaddukte aus Sulfinsäuren, Aldehyden und Aminoverbindungen, wie sie in der DE-A-13 01 566 beschrieben sind, verwendet werden. Durch mehrstündiges Nachheizen der Polymergele im Temperaturbereich 50 bis 130°C, vorzugsweise 70 bis 100°C, können die Qualitätseigenschaften der Polymere noch verbessert werden.

Die erhaltenen Gele werden beispielsweise zu 0 bis 100 mol-%, bevorzugt 25 bis 100 mol-% und besonders bevorzugt zu 50 bis 85 mol-%, bezogen auf eingesetztes Monomer neutralisiert, wobei die üblichen Neutralisationsmittel verwendet werden können, bevorzugt Alkalimetallhydroxide oder -oxide, besonders bevorzugt jedoch Natriumhydroxid, Natriumcarbonat und Natriumhydrogencarbonat.

Üblicherweise wird die Neutralisation durch Einmischung des Neutralisationsmittels als wässrige Lösung oder bevorzugt auch als Feststoff erreicht. Das Gel wird hierzu mechanisch zerkleinert, beispielsweise mittels eines Fleischwolfes und das Neutralisationsmittel wird aufgesprüht, übergestreut oder aufgegossen, und dann sorgfältig untergemischt. Dazu kann die erhaltene Gelmasse noch mehrmals zur Homogenisierung gewolft werden. Die neutralisierte Gelmasse wird dann mit einem Band- oder Walzentrockner getrocknet bis der Restfeuchtegehalt vorzugsweise unter 10 Gew.-%, insbesondere unter 5 Gew.-% liegt. Das getrocknete Hydrogel wird hiernach gemahlen und gesiebt, wobei zur Mahlung üblicherweise Walzenstühle, Stiftmühlen oder Schwingmühlen eingesetzt werden können. Die Partikelgröße des gesiebten Polymeren liegt vorzugsweise im Bereich 45 bis 1000 μm, besonders bevorzugt bei 45 bis 850 μm, ganz besonders bevorzugt bei 100 bis 800 μm und noch mehr bevorzugt bei 100 bis 700 μm. Weiterhin bevorzugt sind Hydrogele, die zu mindestens 97 Gew.% Teilchen mit einem Durchmesser zwischen 150 bis 600 μm enthalten. Besonders bevorzugt sind Hydrogele, die zu mindestens 97 Gew.% Teilchen mit einem Durchmesser zwischen 150 bis 600 μm enthalten und wobei der Anteil der Teilchen mit einem Durchmesser zwischen 500 bis 600 μm nicht mehr als 10 Gew.% beträgt.

Ein weiterer Gegenstand der vorliegenden Erfindung ist ein Herstellverfahren für Hygieneartikel, vorzugsweise Windeln, umfassend die Nachvernetzung wasserabsorbierender Polymere, indem das Polymer mit mindestens einem Nachvernetzer behandelt wird, wobei der mindestens eine Nachvernetzer ein zyklisches Carbamat oder ein zyklischer Harnstoff ist, dadurch gekennzeichnet, dass das zyklische Carbamat oder der zyklische Harnstoff durch Umsetzung eines Aminoalkohols bzw. eines Diamins mit einem zyklischen Karbonat erhalten, aus dem zyklischen Karbonat ein k,m-Diol freigesetzt wurde, wobei gilt m > n und m - n > 1, und das n,m-Diol nach beendeter Reaktion zu höchstens 40% abdestilliert wird.

Der CRC-Wert [g/g] der erfindungsgemäß nachvernetzten wasserabsorbierenden Polymere kann nach den in der Beschreibung angegebenen Methoden gemessen werden und ist bevorzugt größer 15, insbesondere größer 20, besonders bevorzugt größer 25, insbesondere größer 30, insbesondere bevorzugt größer 35.

Der AUL-0,7psi-Wert [g/g] der erfindungsgemäß nachvernetzten wasserabsorbierenden Polymere kann nach den in der Beschreibung angegebenen Methoden gemessen werden und ist bevorzugt größer 10, insbesondere größer 15, besonders bevorzugt größer 20, insbesondere größer 25, insbesondere bevorzugt größer 30.

Der Wert für die extrahierbaren Anteile [Gew.-%] der erfindungsgemäß nachvernetzten wasserabsorbierenden Polymere kann nach den in der Beschreibung angegebenen Methoden gemessen werden und ist bevorzugt kleiner 15, insbesondere kleiner 12, besonders bevorzugt kleiner 10, insbesondere kleiner 8, insbesondere bevorzugt kleiner 7.

Der Wert für die nicht umgesetzten ethylenisch ungesättigten Monomeren [Gew.-ppm] der erfindungsgemäß

nachvernetzten wasserabsorbierenden Polymere kann nach den in der Beschreibung angegebenen Methoden gemessen werden und ist bevorzugt kleiner 500, insbesondere kleiner 400, besonders bevorzugt kleiner 300, insbesondere kleiner 200, insbesondere bevorzugt kleiner 100.

Beispielsweise kann ein erfindungsgemäß hergestellter Hygieneartikel wie folgt aufgebaut sein:

(A) eine obere flüssigkeitsdurchlässige Abdeckung
(B) eine untere flüssigkeitsundurchlässige Schicht
(C) einen zwischen (A) und (B) befindlichen Kern, enthaltend 10 bis 100 Gew.-% des erfindungsgemäßem vernetzten quellbaren hydrogelbildenden Polymers

0 bis 90 Gew.-% hydrophiles Fasermaterial

bevorzugt 30 bis 100 Gew.-% des erfindungsgemäß vernetzten quellbaren hydrogelbildenden Polymers, 0 bis 70 Gew.-% hydrophiles Fasermaterial besonders bevorzugt 50 bis 100 Gew.-% des erfindungsgemäß vernetzten quellbaren hydrogelbildenden Polymers 0 bis 50 Gew.-% hydrophiles Fasermaterial
insbesondere bevorzugt 70 bis 100 Gew.-% des erfindungsgemäß vernetzten quellbaren hydrogelbildenden Polymers, 0 bis 30 Gew.-% hydrophiles Fasermaterial
am meisten bevorzugt 90 bis 100 Gew.-% des erfindungsgemäß vernetzten quellbaren hydrogelbildenden Polymers, 0 bis 10 Gew.-% hydrophiles Fasermaterial

(D) gegebenenfalls eine sich unmittelbar oberhalb und unterhalb des Kerns (C) sich befindende Tissueschicht und
(E) gegebenenfalls eine zwischen (A) und (C) sich befindende Aufnahmeschicht.

Unter Hygieneartikel sind dabei beispielsweise Inkontinenzeinlagen und Inkontinenzhosen für Erwachsene oder Windeln für Babys zu verstehen.

Bei der flüssigkeitsdurchlässigen Abdeckung (A) handelt es sich um die Schicht, die direkten Hautkontakt hat. Das Material hierfür besteht hierbei aus üblichen synthetischen oder halbsynthetischen Fasern oder Filme von Polyester, Polyolefine, Rayon oder natürlichen Fasern wie Baumwolle. Bei nichtgewebten Materialien sind die Fasern in der Regel durch Bindemittel wie Polyacrylate zu verbinden. Bevorzugte Materialien sind Polyester, Rayon und deren Blends, Polyethylen und Polypropylen. Beispiele für flüssigkeitsdurchlässige Schichten sind beschrieben in WO 99/57355, EP-A-1 023 883.

Die flüssigkeitsundurchlässige Schicht (B) besteht in der Regel aus einer Folie aus Polyethylen oder Polypropylen.

Der Kern (C) enthält neben dem erfindungsgemäßen vernetzten quellbaren hydrogelbildenden Polymer hydrophiles Fasermaterial. Unter hydrophil ist zu verstehen, dass sich wässrige Flüssigkeiten schnell über die Faser verteilen.

Für gewöhnlich ist das Fasermaterial Cellulose, modifizierte Cellulose, Rayon, Polyester wie Polyethylenterephthalat. Besonders bevorzugt werden Cellulosefasern wie Zellstoff. Die Fasern haben in der Regel einen Durchmesser von 1 bis 200 μm, bevorzugt 10 bis 100 μm. Darüberhinaus haben die Fasern eine Mindestlänge von 1 mm.

Der Aufbau und die Form von Windeln ist allgemein bekannt und beispielsweise in der WO 95/26209 Seite 66, Zeile 34 bis Seite 69, Zeile 11, WO-A-03/104300, DE-A-196 04 601, EP-A-0 316 518 und EP-A-0 202 127 beschrieben.

Zur Bestimmung der Güte der Oberflächennachvernetzung wird das getrocknete Hydrogel mit den Testmethoden geprüft, die nachfolgend beschrieben sind:

Methoden:

<u>Zentrifugenretentionskapazität (CRC Centrifuge Retention Capacity)</u>

Bei dieser Methode wird die freie Quellbarkeit des wasserabsorbierenden Polymeren im Teebeutel bestimmt. Zur Bestimmung der CRC werden 0,2000 ± 0,0050 g getrocknetes Polymer in einem 60 x 85 mm großen Teebeutel eingewogen, der anschließend verschweißt wird. Der Teebeutel wird für 30 Minuten in einen Überschuss von 0,9 gew.-%iger Kochsalzlösung gegeben (mindestens 0,83 l Kochsalzlösung/1 g Polymerpulver). Anschließend wird der Teebeutel 3 Minuten lang bei 250 G zentrifugiert. Die Bestimmung der vom Hydrogel festgehaltenen Flüssigkeitsmenge geschieht durch Auswägen des zentrifugierten Teebeutels.

Die Zentrifugenretentionskapazität kann auch nach der von der EDANA (European Disposables and Nonwovens Association) empfohlenen Testmethode Nr. 441.2-02 "Centrifuge retention capacity" bestimmt werden.

Absorption unter Druck (AUL Absorbency Under Load) 0,7 psi (4830 Pa)

Die Messzelle zur Bestimmung der AUL 0,7 psi ist ein Plexiglas-Zylinder mit einem Innendurchmesser von 60 mm und einer Höhe von 50 mm, der an der Unterseite einen angeklebten Edelstahl-Siebboden mit einer Maschenweite von 36 μm besitzt. Zu der Messzelle gehört weiterhin eine Plastikplatte mit einem Durchmesser von 59 mm und ein Gewicht, welches zusammen mit der Plastikplatte in die Messzelle hineingestellt werden kann. Das Gewicht der Plastikplatte und das Gewicht betragen zusammen 1344 g. Zur Durchführung der Bestimmung der AUL 0,7 psi wird das Gewicht des leeren Plexiglas-Zylinders und der Plastikplatte ermittelt und als $W_0$ notiert. Dann werden 0,900 ± 0,005 g getrocknetes Polymer in den Plexiglas-Zylinder eingewogen und möglichst gleichmäßig auf dem Edelstahl-Siebboden verteilt. Anschließend wird die Plastikplatte vorsichtig in den Plexiglas-Zylinder hineingelegt und die gesamte Einheit gewogen; das Gewicht wird als $W_a$ notiert. Nun wird das Gewicht auf die Plastikplatte in dem Plexiglas-Zylinder gestellt.

In die Mitte der Petrischale mit einem Durchmesser von 200 mm und einer Höhe von 30 mm wird eine keramische Filterplatte mit einem Durchmesser von 120 mm, einer Höhe von 10 mm und einer Porosität 0 gelegt und soviel 0,9 gew.-%ige Natriumchloridlösung eingefüllt, dass die Flüssigkeitsoberfläche mit der Filterplattenoberfläche abschließt, ohne dass die Oberfläche der Filterplatte benetzt wird. Anschließend wird ein rundes Filterpapier mit einem Durchmesser von 90 mm und einer Porengröße < 20 μm (S&S 589 Schwarzband von Schleicher & Schüll) auf die keramische Platte gelegt. Der wasserabsorbierendes Polymer enthaltende Plexiglas-Zylinder wird mit Plastikplatte und Gewicht nun auf das Filterpapier gestellt und dort für 60 Minuten belassen. Nach dieser Zeit wird die komplette Einheit aus der Petrischale vom Filterpapier herausgenommen und anschließend das Gewicht aus dem Plexiglas-Zylinder entfernt. Der gequollenes Hydrogel enthaltende Plexiglas-Zylinder wird zusammen mit der Plastikplatte ausgewogen und das Gewicht als $W_b$ notiert.

Die Absorption unter Druck (AUL) wird wie folgt berechnet:

$$\text{AUL 0,7 psi [g/g]} = [W_b - W_a]/[W_a - W_0]$$

Die Absorption unter Druck kann auch nach der von der EDANA (European Disposables and Nonwovens Association) empfohlenen Testmethode Nr. 442.2-02 "Absorption under pressure" bestimmt werden.

Absorption unter Druck (AUL Absorbency Under Load) 0,3 psi (2070 Pa)

Die Messung wird analog der AUL 0.3 psi durchgeführt. Das Gewicht der Plastikplatte und des Gewichts betragen zusammen 576 g.

Extrahierbare 16h

Der Gehalt an extrahierbaren Bestandteilen des wassrabsorbierenden Polymeren kann nach der von der EDANA (European Disposables and Nonwovens Association) empfohlenen Testmethode Nr. 470.2-02 "Determination of extractable polymer content by potentiometric titration" bestimmt werden.

Oberflächenspannung des wässrigen Extraktes

Es werden 0,50 g getrocknetes Polymer in ein kleines Becherglas eingewogen und mit 40 ml einer 0,9 gew.%-igen Kochsalzlösung versetzt. Der Inhalt des Becherglases wird 3 Minuten bei 500 U/m mit einem Magnetrührstab gerührt, dann lässt man 2 Minuten absitzen. Schliesslich wird die Oberflächenspannung der überstehenden wässrigen Phase mit einem Digital-Tensiometer K10-ST oder einem vergleichbaren Gerät mit Platinplatte gemessen (Fa. Kruess).

Restmonomere

Der Gehalt an nicht umgesetzten ethylenisch ungesättigten Monomeren im wasserabsorbierenden Polymeren kann nach der von der EDANA (European Disposables and Nonwovens Association) empfohlenen Testmethode Nr. 410.2-02 "Residual monomers" bestimmt werden.

Flüssigkeitsweiterleitung (SFC Saline Flow Conductivity)

Die Flüssigkeitsweiterleitung einer gequollenen Gelschicht unter Druckbelastung von 0,3 psi (2070 Pa) wird, wie in EP-A-0 640 330 beschrieben, als Gel-Layer-Permeability einer gequollenen Gelschicht aus superabsorbierendem

Polymer bestimmt, wobei die in zuvor genannter Patentanmeldung auf Seite 19 und in Figur 8 beschriebene Apparatur dahingehend modifiziert wurde, dass die Glasfritte (40) nicht mehr verwendet wird, der Stempel (39) aus gleichem Kunststoffmaterial besteht wie der Zylinder (37) und jetzt über die gesamte Auflagefläche gleichmäßig verteilt 21 gleichgroße Bohrungen enthält. Die Vorgehensweise sowie Auswertung der Messung bleibt unverändert gegenüber EP-A-0 640 330. Der Durchfluss wird automatisch erfasst.

Die Flüssigkeitsweiterleitung (SFC) wird wie folgt berechnet:

$$SFC\ [cm^3 s/g] = (F_g(t{=}0) x L_0)/(d x A x WP),$$

wobei $F_g(t{=}0)$ der Durchfluss an NaCl-Lösung in g/s ist, der anhand einer linearen Regressionsanalyse der Daten $F_g(t)$ der Durchflussbestimmungen durch Extrapolation gegen t=0 erhalten wird, $L_0$ die Dicke der Gelschicht in cm, d die Dichte der NaCl-Lösung in g/cm$^3$, A die Fläche der Gelschicht in cm$^2$ und WP der hydrostatische Druck über der Gelschicht in dyn/cm$^2$ darstellt.

<u>Farbzahl</u>

Die Farbzahl kann nach ASTM D 1209 gemessen werden.

<u>Geruchstest</u>

Zur Beurteilung des Geruchs des angequollenen wasserabsorbierenden Polymeren werden 2,0 g trockenen Hydrogels in ein 50 ml Becherglas eingewogen. Dann werden 20 g 0,9 gew-%ige Kochsalzlösung bei 23°C zugegeben. Das Becherglas mit dem quellenden wasserabsorbierenden Polymeren wird gasdicht verschlossen und 3 Minuten stehen gelassen. Danach wird der Verschluß entfernt und der Geruch beurteilt. Jede Probe wird dabei von mindestens 3 Versuchspersonen geprüft, wobei jeweils eine separate Probe hergestellt wird.

Beispiele

Beispiel 1 (Vergleich):

Herstellung von N-(2-Hydroxyethyl)-oxazolidin-2-on als Lösung in Propylenglykol

Unter Stickstoffatmosphäre wurden 5,1 mol Propylenkarbonat bei 23°C vorgelegt. Innerhalb von 30 bis 120 Minuten wurden 5 mol Diethanolamin unter Rühren zudosiert, wobei die Temperatur der Reaktionsmischung anstieg. Nach beendeter Zugabe heizte man die Mischung innerhalb von 10 bis120 Minuten auf 120°C auf und ließ bei dieser Temperatur 12 Stunden lang rühren. Diethanolamin wurde zu >98% umgesetzt, die erhaltene Lösung enthielt 60 bis 63gew.-% N-(2-Hydroxyethyl)-oxazolidin-2-on.

Beispiel 2 (Vergleich):

Herstellung von N-(2-Hydroxyethyl)-oxazolidin-2-on als Lösung in Propylenglykol mit anschließender Destillation

Unter Stickstoffatmosphäre wurden 5,1 mol Propylenkarbonat bei 23°C vorgelegt. Innerhalb von 30 bis 120 Minuten wurden 5 mol Diethanolamin unter Rühren zudosiert, wobei die Temperatur der Reaktionsmischung anstieg. Nach beendeter Zugabe heizte man die Mischung innerhalb von 10 bis 120 Minuten auf 120°C auf und ließ bei dieser Temperatur 2 Stunden lang rühren. Anschließend wurde ein Vakuum von 10 bis 20 mbar angelegt, die Temperatur von 120°C auf 160°C erhöht und Propylenglykol abdestilliert. Das als undestilliertes Sumpfprodukt nahezu quantitativ erhaltene N-(2-Hydroxyethyl)-oxazolidin-2-on enthielt 96 bis 98 Gew.-% Wertprodukt und hatte eine Farbzahl von 0 bis 15 nach Hazen.

Beispiel 3 (Vergleich):

Herstellung von N-(2-Hydroxyethyl)-oxazolidin-2-on als Lösung in Propylenglykol unter NaOH-Katalyse

Unter Stickstoffatmosphäre wurden 5,1 mol Propylenkarbonat bei 23°C vorgelegt. Innerhalb von 30 bis 120 Minuten wurden 5 mol Diethanolamin unter Rühren zudosiert, wobei die Temperatur der Reaktionsmischung anstieg.

Nach beendeter Zugabe heizte man die Mischung innerhalb von 10 bis 120 Minuten auf 100°C auf. Man gab bei dieser Temperatur 2,5 mol-% NaOH in fester Form zu und ließ bei dieser Temperatur 2 Stunden lang rühren. Diethanolamin wurde zu >98% umgesetzt, die erhaltene Lösung enthielt 60 bis 63 gew.-% N-(2-Hydroxyethyl)-oxazolidin-2-on. Das Wertprodukt hatte eine Farbzahl von 0 bis 5 nach Hazen.

Beispiel 4 (Vergleich):

Herstellung von N-(2-Hydroxyethyl)-oxazolidin-2-on als Lösung in Propylenglykol unter NaOH-Katalyse mit anschließender Destillation

Unter Stickstoffatmosphäre wurden 5,1 mol Propylenkarbonat bei 23°C vorgelegt. Innerhalb von 30 bis 120 Minuten wurden 5 mol Diethanolamin unter Rühren zudosiert, wobei die Temperatur der Reaktionsmischung anstieg. Nach beendeter Zugabe heizte man die Mischung innerhalb von 10 bis 120 Minuten auf 100°C auf. Man gab bei dieser Temperatur 2,5 mol-% NaOH in fester Form zu und ließ bei dieser Temperatur 2 Stunden lang rühren. Anschließend wurde ein Vakuum von 10 bis 20mbar angelegt, die Temperatur von 100°C auf 160°C erhöht und Propylenglykol abdestilliert. Das als undestilliertes Sumpfprodukt nahezu quantitativ erhaltene N-(2-Hydroxyethyl)-oxazolidin-2-on enthielt 96-98% Wertprodukt und hatte eine Farbzahl von 0 bis 25 nach Hazen.

Beispiel 5:

Herstellung von N-(2-Hydroxyethyl)-oxazolidin-2-on als Lösung in Propan-1,3-diol

Analog zu Beispiel 1, wobei 1,3-Dioxan-2-on statt Propylenkarbonat eingesetzt wurde. Diethanolamin wurde zu >98% umgesetzt.

Beispiel 6:

Herstellung von N-(2-Hydroxyethyl)-oxazolidin-2-on als Lösung in Propan-1,3-diol unter NaOH-Katalyse

Analog zu Beispiel 3, wobei 1,3-Dioxan-2-on statt Propylenkarbonat eingesetzt wurde. Diethanolamin wurde zu >98% umgesetzt.

Beispiele zur Herstellung von Superabsorbern:

Beispiel 7 (Referenz):

Herstellung Grundpolymer

In einen Lödige-Pflugscharkneter Typ VT 5R-MK (5 l Volumen) wurden 460 g entionisiertes Wasser, 213,9 g Acrylsäure, 1924,9 g einer 37,3 gew.-%igen Natriumacrylatlösung (100 mol-% neutralisiert) sowie 2,29 g des Vernetzers Glycerin-3 EO-Triacrylat (Triacrylat des insgesamt 3-fach ethoxylierten Glycerins) vorgelegt und unter Durchperlen von Stickstoff 20 Minuten inertisiert. Dann wurde durch Zusatz (verdünnte wässrige Lösungen) von 2,139 g Natriumpersulfat gelöst in 12,12 g Wasser, 0,046 g Ascorbinsäure gelöst in 9,12 g Wasser sowie 0,127 g 30 gew.-%iges Wasserstoffperoxid gelöst in 1,15 g Wasser bei ca. 20 °C gestartet. Nach dem Start wurde die Temperatur des Heizmantels der Reaktionstemperatur im Reaktor mittels Regelung nachgeführt. Das letztlich erhaltene krümelige Gel wurde dann bei 160°C ca. 3 Stunden im Umlufttrockenschrank getrocknet.

Das getrocknete Grundpolymer wurde gemahlen und auf 150 bis 850 μm abgesiebt.

Das Grundpolymer hatte folgende Eigenschaften:

CRC = 34 g/g
AUL 0.3 psi = 13 g/g
Extrahierbare 16h = 8,1 Gew.%
Restfeuchte = 1,9 Gew.%
Oberflächenspannung des wässrigen Extrakts = 0,0715 N/m

Beispiele 8 bis 11 (Vergleich) und 12 - 14:

Nachvernetzung des Grundpolymers aus Beispiel 7

20 g Polymer (auf 300 bis 850 μm abgesiebt) aus Beispiel 7 wurden in einem Waring-Labormischer vorgelegt, der mit einem Aufsatz mit stumpfen Mischklingen ausgerüstet war. Bei mittlerer Drehzahl wurden dann mittels einer Injektionsspritze langsam durch ein Loch im Deckel des Mischaufsatzes genau 0,80 g der Nachvernetzungslösung langsam und unter Rühren zugefügt, um das Polymer möglichst gleichmäßig zu benetzen.

Die Nachvernetzungslösung hatte die in der Tabelle 1 angegebene Zusammensetzung. Eingesetzt wurde jeweils das in den Herstellbeispielen beschriebene Endprodukt.

Tab. 1: Nachvernetzungslösungen

| Beispiel | Isopropanol* | Wasser* | Nachvernetzungsmittel* |
|---|---|---|---|
| 8 **) | 1,146 | 2,674 | 0,18 (Bsp. 3) |
| 9 **) | 1,170 | 2,730 | 0,10 (Bsp. 4) |
| 10 **) | 1,146 | 2,674 | 0,18 (Bsp. 1) |
| 11 **) | 1,170 | 2,730 | 0,10 (Bsp. 2) |
| 12 | 1,146 | 2,674 | 0,18 (Bsp. 6) |
| 13 | 1,170 | 2,730 | 0,10 (Bsp. 6) |
| 14 | 1,146 | 2,674 | 0,18 (Bsp. 5) |
| *) Alle Mengenangaben in Gew.% bezogen auf eingesetztes Polymer.<br>**) Vergleich | | | |

Das befeuchtete Polymer wurde durch kurzes Rühren im Mischer homogenisiert und dann auf einem Uhrglas im Umlufttrockenschrank für 60 Minuten bei 175°C getempert. Anschließend wurde durch ein 850 μm Sieb gesiebt, um gebildete Agglomerate zu entfernen.

Die Eigenschaften des nachvernetzten Polymers sind in Tabelle 2 aufgelistet.

Tab. 2 nachvernetzte Polymere

| Bsp. | CR C [g/g ] | AUL 0.7 [g/g] | Extrah. 16h [Gew.%] | SFC [$10^{-7}$ $cm^3s/g$] | Oberflächenspannung des wässr. Extrakts [N/m] | Geruch beim Befeuchten |
|---|---|---|---|---|---|---|
| 8 **) | 27, 5 | 22,9 | 7,2 | 105 | 0,072 | schwach alkoholisch |
| 9 **) | 27, 9 | 23,0 | 7,1 | 100 | 0,072 | fast geruchlos |
| 10 **) | 27, 4 | 23,1 | 6,9 | 103 | 0,072 | schwach alkoholisch |
| 11 **) | 28, 0 | 23,0 | 7,2 | 107 | 0,072 | fast geruchlos |
| 12 | 26, 6 | 22,0 | 7,3 | 113 | 0,072 | sehr schwach |
| 13 | 27, 0 | 22,7 | 7,3 | 114 | 0,073 | sehr schwach |
| 14 | 26, 8 | 22,2 | 7,0 | 110 | 0,072 | sehr schwach |
| *) Alle Mengenangaben in Gew.% bezogen auf eingesetztes Polymer.<br>**) Vergleich | | | | | | |

## Patentansprüche

1. Verfahren zur Nachvernetzung wasserabsorbierender Polymere, indem das Polymer mit mindestens einem Nachvernetzer behandelt und während oder nach dem Behandeln durch Temperaturerhöhung nachvernetzt und getrocknet wird, wobei der mindestens eine Nachvernetzer ein zyklisches Carbamat oder ein zyklischer Harnstoff ist, **dadurch gekennzeichnet, dass** das zyklische Carbamat oder der zyklische Harnstoff durch Umsetzung eines Aminoalkohols bzw. eines Diamins mit einem zyklischen Karbonat erhalten, aus dem zyklischen Karbonat ein n,m-Diol freigesetzt wurde, wobei gilt m > n und m-n > 1, und das n,m-Diol nach beendeter Reaktion zu höchstens 40% abdestilliert wird.

2. Verfahren nach Anspruch 1, **dadurch gekennzeichnet, dass** das mit mindestens einem Nachvernetzer behandelte Polymer während oder nach dem Behandeln durch Temperaturerhöhung nachvernetzt wird.

3. Verfahren nach Anspruch 1 oder 2, **dadurch gekennzeichnet, dass** das mit mindestens einem Nachvernetzer behandelte Polymer während oder nach dem Behandeln getrocknet wird.

4. Verfahren nach einem der Ansprüche 1 bis 3, **dadurch gekennzeichnet, dass** der Nachvernetzer ein zyklisches Carbamat ist und zusammen mit dem n,m-Diol eingesetzt wird.

5. Verfahren nach Anspruch 4, **dadurch gekennzeichnet, dass** das Molverhältnis von n,m-Diol zu zyklischem Carbamat von 1,1:1 bis 1:2,5 beträgt.

6. Verfahren nach einem der Ansprüche 1 bis 5, **dadurch gekennzeichnet, dass** das zyklische Carbamat oder der zyklische Harnstoff am Stickstoffatom eine Hydroxyalkyl-Gruppe aufweist.

7. Verfahren nach einem der Ansprüche 1 bis 6, **dadurch gekennzeichnet, dass** das zyklische Carbamat oder der zyklische Harnstoff zusammen mit einem zyklischen Karbonat eingesetzt wird.

8. Verfahren nach einem der Ansprüche 1 bis 7, **dadurch gekennzeichnet, dass** bei der Umsetzung des Aminoalkohols mit dem zyklischen Karbonat ein basischer Katalysator verwendet wird.

9. Verfahren nach Anspruch 8, **dadurch gekennzeichnet, dass** der basische Katalysator Natriumhydroxid ist.

10. Verfahren zur Herstellung von Hygieneartikeln, umfassend eine Nachvernetzung eines wasserabsorbierenden Polymeren gemäß einem Verfahren der Ansprüche 1 bis 9.

## Claims

1. A process for postcrosslinking a water absorbing polymer by the polymer being treated with at least one postcrosslinker and postcrosslinked and dried during or after the treatment by raising the temperature, the at least one postcrosslinker being a cyclic carbamate or a cyclic urea, wherein the cyclic carbamate or the cyclic urea was obtained by reacting respectively an aminoalcohol or a diamine with a cyclic carbonate, an n,m-diol was freed from the cyclic carbonate where m > n and m-n > 1, and the n,m diol is distilled off to not more than 40% after the reaction has ended.

2. The process according to claim 1 wherein the polymer treated with at least one postcrosslinker is postcrosslinked during or after the treatment by raising the temperature.

3. The process according to claim 1 or 2 wherein the polymer treated with at least one postcrosslinker is dried during or after the treatment.

4. The process according to any one of claims 1 to 3 wherein the postcrosslinker is a cyclic carbamate and is used together with the n,m-diol.

5. The process according to claim 4 wherein the molar ratio of n,m-diol to cyclic carbamate is in the range from 1.1:1 to 1:2.5.

6. The process according to any one of claims 1 to 5 wherein the cyclic carbamate or the cyclic urea has a hydroxyalkyl group on the nitrogen atom.

7. The process according to any one of claims 1 to 6 wherein the cyclic carbamate or the cyclic urea is used together

with a cyclic carbonate.

8. The process according to any one of claims 1 to 7 wherein a basic catalyst is used in the reaction of the aminoalcohol with the cyclic carbonate.

9. The process according to claim 8 wherein the basic catalyst is sodium hydroxide.

10. A process for producing a hygiene article comprising a postcrosslinking of a water absorbing polymer according to a process of claims 1 to 9.

## Revendications

1. Procédé de post-réticulation de polymères absorbant l'eau, selon lequel le polymère est traité avec au moins un agent de post-réticulation, et post-réticulé et séché pendant ou après le traitement par élévation de la température, ledit au moins un agent de post-réticulation étant un carbamate cyclique ou une urée cyclique, **caractérisé en ce que** le carbamate cyclique ou l'urée cyclique est obtenu par mise en réaction d'un aminoalcool ou d'une diamine avec un carbonate cyclique, un n,m-diol étant libéré à partir du carbonate cyclique, avec m > n et m-n > 1, et le n,m-diol étant éliminé par distillation à hauteur d'au plus 40 % après la fin de la réaction.

2. Procédé selon la revendication 1, **caractérisé en ce que** le polymère traité avec au moins un agent de post-réticulation est post-réticulé pendant ou après le traitement par élévation de la température.

3. Procédé selon la revendication 1 ou 2, **caractérisé en ce que** le polymère traité avec au moins un agent de post-réticulation est séché pendant ou après le traitement.

4. Procédé selon l'une quelconque des revendications 1 à 3, **caractérisé en ce que** l'agent de post-réticulation est un carbamate cyclique et est utilisé conjointement avec le n,m-diol.

5. Procédé selon la revendication 4, **caractérisé en ce que** le rapport molaire entre le n,m-diol et le carbamate cyclique est de 1,1:1 à 1:2,5.

6. Procédé selon l'une quelconque des revendications 1 à 5, **caractérisé en ce que** le carbamate cyclique ou l'urée cyclique comprend un groupe hydroxyalkyle sur l'atome d'azote.

7. Procédé selon l'une quelconque des revendications 1 à 6, **caractérisé en ce que** le carbamate cyclique ou l'urée cyclique est utilisé conjointement avec un carbonate cyclique.

8. Procédé selon l'une quelconque des revendications 1 à 7, **caractérisé en ce qu'**un catalyseur basique est utilisé lors de la mise en réaction de l'aminoalcool avec le carbonate cyclique.

9. Procédé selon la revendication 8, **caractérisé en ce que** le catalyseur basique est l'hydroxyde de sodium.

10. Procédé de fabrication d'articles d'hygiène, comprenant une post-réticulation d'un polymère absorbant l'eau par un procédé selon les revendications 1 à 9.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0083022 A
- EP 0543303 A
- EP 0530438 A
- EP 0349935 A
- US 4666983 A
- US 5385983 A
- DE 19807502 A
- WO 03031482 A
- DE 198545732 A
- DE 19807992 A
- DE 10204937
- US 2437390 A
- US 4933462 A
- EP 0900825 A
- US 2755286 A
- JP 61007260 A
- JP 60097967 A
- JP 60152476 A
- JP 2002105064 A
- US 4286082 A
- DE 2706135 C
- US 4340706 A
- DE 3713601 C
- DE 2840010 C
- DE 4344548 A
- DE 4020780 A
- DE 4015085 A
- DE 3917846 A
- DE 3807289 A
- DE 3533337 A
- DE 3503458 A
- DE 4244548 A
- DE 4219607 A
- DE 4021847 A
- DE 3831261 A
- DE 3511086 A
- DE 3118172 A
- DE 3028043 A
- DE 4418881 A
- EP 0801483 A
- EP 0455985 A
- EP 0467073 A
- EP 0312952 A
- EP 0205874 A
- EP 0499774 A
- DE 2612846 A
- EP 0205674 A
- US 5145906 A
- EP 0670073 A
- US 4057521 A
- US 4062817 A
- US 4525527 A
- US 4295987 A
- US 5011892 A
- US 4076663 A
- US 4931497 A
- US 5041496 A
- EP 0343427 A
- WO 03104301 A1
- WO 0138402 A
- EP 0955086 A
- DE 1301566 A
- WO 9957355 A
- EP 1023883 A
- WO 9526209 A
- WO 03104300 A
- DE 19604601 A
- EP 0316518 A
- EP 0202127 A
- EP 0640330 A


### In der Beschreibung aufgeführte Nicht-Patentliteratur


- *Tetrahedron Letters,* 1974, 2899-2900
- *Journal of Organic Chemistry,* 1961, 3495-3498
- *Nippon Kagaku Kaish,* 1974, vol. 8, 1592-1594
- **F.L. BUCHHOLZ ; A.T. GRAHAM.** Modern Superabsorbent Polymer Technology. Wiley-VCH, 1998, 77-84